**Europäisches Patentamt**

**European Patent Office**

**Office européen des brevets**

(19)

(11) Veröffentlichungsnummer: **0 254 092**
**A1**

(12) # EUROPÄISCHE PATENTANMELDUNG

(21) Anmeldenummer: **87109501.4**

(22) Anmeldetag: **02.07.87**

(51) Int. Cl.⁴: **C07D 307/60** , **C07C 51/215**

(30) Priorität: **19.07.86 DE 3624439**

(43) Veröffentlichungstag der Anmeldung:
**27.01.88 Patentblatt 88/04**

(84) Benannte Vertragsstaaten:
**BE DE FR GB NL**

(71) Anmelder: **BASF Aktiengesellschaft**
**Carl-Bosch-Strasse 38**
**D-6700 Ludwigshafen(DE)**

(72) Erfinder: **Kotter, Michael, Dr.**
**Fruehmessweinberg 22**
**D-7320 Bruchsal(DE)**
Erfinder: **Riekert, Lothar, Prof. Dr.**
**Im Eichbaeumle 21**
**D-7500 Karlsruhe(DE)**

(54) **Verfahren zur Herstellung von Maleinsäurenhydrid durch partielle Oxidation.**

(57) Die Anmeldung betrifft ein Verfahren zur Herstellung von Maleinsäureanhydrid durch partielle Oxidation von $C_4$-Kohlenwasserstoffen wie 1-Buten, 2-Buten und Butan oder Gemischen derselben in der Gasphase in Gegenwart von Katalysatoren, die durch Imprägnieren chemisch inerter, abriebfester Trägerstoffe mit einer alkoholischen Lösung von Vanadinoxiden und/oder Estern der Vanadinsäure und/oder Alkoxiden des Vanadiums und/oder Vanadylalkoholaten und Phosphorsäureestern behandelt und darauf vor der üblichen Trocknung und Calzinierung der Einwirkung von Wasserdampf ausgesetzt worden sind.

EP 0 254 092 A1

## Verfahren zur Herstellung von Maleinsäureanhydrid durch partielle Oxidation

Es ist bekannt, Maleinsäureanhydrid (MSA) durch partielle Oxidation von $C_4$-Kohlenwasserstoffen wie 1-Buten, 2-Buten und Butan an Katalysatoren auf der Basis von Vanadin/Phosphor-Mischoxiden, die gegebenenfalls durch oxidische Zusätze des Titans, Molybdäns und Kobalts modifiziert sind, herzustellen.

Die Herstellung der Katalysatoren erfolgt üblicherweise durch Fällung einer Vorstufe der katalytisch wirksamen Mischoxide aus vanadinhaltigen wäßrigen Lösungen oder alkoholischer Suspension (US-PS 4 225 465). Die Weiterverarbeitung des durch Filtration abgetrennten, pulverförmigen Katalysatorvorläufers zu mechanisch langzeitstabilen Pellets, Tabletten und Extrudaten bereitet jedoch Schwierigkeiten, die u.a. durch die quellfähige Schichtstruktur des Vorläufers bedingt sind.

Es war daher die Aufgabe gestellt, mechanisch stabile und abriebfeste Katalysatorformkörper, die auch für die Verwendung in der Wirbelschicht geeignet sind, durch Aufbringung der katalytisch wirksamen Komponente auf einen vorgeformten Katalysatorträger herzustellen und für die Umsetzung zu verwenden. Dabei war darauf zu achten, daß der Träger selbst keine unerwünschte katalytische Wirksamkeit bei der Oxidation besitzt.

Es wurde nun gefunden, daß man gute Ergebnisse erzielt, wenn man bei der Herstellung von Maleinsäureanhydrid durch partielle Oxidation von $C_4$-Kohlenwasserstoffen wie 1-Buten, 2-Buten und Butan oder Gemischen derselben in der Gasphase in Gegenwart von Phosphor-Vanadium-Sauerstoffkatalysatoren Katalysatoren verwendet, die zuerst durch Imprägnieren chemisch inerter, abriebfester Trägerstoffe mit einer alkoholischen Lösung von Vanadinoxiden, Estern der Vanadinsäure und/oder Alkoxiden des Vanadiums und/oder Vanadylalkoholaten und Phosphorsäureestern behandelt und darauf vor der üblichen Trocknung und Calcinierung der Einwirkung von Wasserdampf ausgesetzt worden sind.

Für die erfindungsgemäße Umsetzung haben sich als abriebfeste Trägerstoffe auf die die katalytisch aktiven Komponenten durch Imprägnieren oder Tränken aufgetragen werden, die verschiedene Silikate des Aluminiums und Magnesiums, z.B. Steatit und Magnesiumaluminiumsilikate, Aluminiumoxid z.B. Korund als geeignet erwiesen. Insbesondere für die Verwendung der Katalysatoren in der Wirbelschicht ist Cordierit ein geeignetes Trägermaterial. Die Katalysatoren können in der üblichen Form z.B. als Stränge, Kugeln, Ringe oder sternförmige Katalysatorkörper verwendet werden. Besonders geeignet sind auch Wabenkörper.

Die partielle Oxidation von $C_4$-Kohlenwasserstoffen wie 1-Buten, 2-Buten und Butan oder Gemischen derselben wird normalerweise drucklos bei Temperaturen von 300 - 550°C im Festbett oder in der Wirbelschicht ausgeführt.

Für die Herstellung der alkoholischen Lösung von Vanadinoxid und/oder Estern der Vanadinsäure und/oder Alkoxiden des Vanadiums und/oder Vanadylalkoholaten und Phosphorsäureestern sind alle Alkohole, die unter normalen Bedingungen flüssig sind und in denen die verwendeten Bestandteile gut löslich sind, geeignet; z.B. aliphatische oder cycloaliphatische ein-und zweiwertige Alkohole, wie Alkanole, z.B. Propanol und Butanol, insbesondere Isopropanol und Isobutanol. Man verwendet für diese Lösung Vanadinoxide und Ester der Vanadinsäure bzw. Vanadylalkoholate oder Alkoxide des Vanadiums z.B. $VO(OR)_3$, $VO(OC_3H_7)_3$, die in technisch günstigen Zeiten nach dem beschriebenen Vefahren vollständig hydrolisiert werden können, z.B. neben Vanadinoxid, die Ester der Vanadinsäure, Vanadylakoholate mit Ethanol, Propanol oder Butanol und als Phosphorsäureester z.B. die Ethylester, Propylester, Butylesterverbindungen der allgemeinen Formel $PO(OR)_3$, z.B. $PO(OC_3H_7)_3$, der Ortho-und Methaphosphorsäure. Besonders geeignet ist als Imprägnierlösung eine alkoholische Lösung von Vanadisopropylat und Phosphorsäuredibutylester. Der Katalysatorvorläufer wird in der Porenstruktur durch gleichzeitige Hydrolyse des Alkoxids und des Esters gebildet. Die autokatalytische Simultanhydrolyse der beiden Komponenten läßt sich in den Poren des Trägers auslösen, in dem man der Imprägnierlösung etwas Wasser in der zur vollständigen Hydrolyse nicht ausreichenden Menge zusetzt und den damit imprägnierten Träger in einer wasserdampfhaltigen Atmosphäre erwärmt. Das zur vollständigen Hydrolyse noch erforderliche Wasser wird dann aus der Gasphase aufgenommen.

Bei dem erfindungsgemäßen Verfahren erzielt man Selektivitäten in bezug auf die Herstellung von MSA von etwa 50 bis 55 Mol.-% an mechanisch stabilen Tränkkontakten.

Die Tränkkatalysatoren können auch vor der Trocknung und Calcinierung mit verschiedenen anderen Elementen oder Verbindungen, z.B. durch Zusatz von Promotoren verbessert werden. Als Promotoren sind z.B. geeignet: die Oxide des Titans, Molybdäns oder Kobalts.

Der Vorteil des erfindungsgemäßen Verfahrens unter Verwendung der durch Imprägnieren von chemisch inerten abriebfesten Trägerstoffen hergestellten Oxidationskatalysatoren im Gegensatz zu den bisher verwendeten Kata lysatoren, insbesondere den Fällungskatalysatoren besteht darin, daß man bei gleichguten Selektivitäten durch erhöhte Abriebfestigkeit wesentlich längere Standzeiten erzielt und außerdem durch das Imprägnierungs-bzw. Tränkverfahren eine gleichmäßigere Katalysatorqualität.

Beispiel

10 g eines wabenförmigen Trägers aus Cordierit, dessen Wabenkörper etwa 1 mm Kanaldurchmesser bei einer Wandstärke der Zwischenwände von 0,2 mm wird in eine Lösung von 30 g Isopropanol, 0,45 g Wasser, 7,3 g Vanadylisopropylat und 6,3 g Phosphorsäuredibutylester getaucht, danach von der Lösung getrennt und der imprägnierte Träger während 4 Stunden in einer wasserdampfgesättigten Atmosphäre bei 80°C belassen, bevor derselbe bei 120°C getrocknet und bei 450°C 2 Stunden kalziniert wird.

Über 0,5 g eines nach dieser Vorschrift hergestellten Katalysators wird ein Reaktionsgemisch von 1,69 Mol.-% 1-Buten in Luft bei 450°C geleitet. Bei einem Umsatz von 90 % und einer Raumgeschwindigkeit 7 $m^3/kg^{-1}h^{-1}$ erhält man nach einer Laufzeit von 48 Stunden ein Reaktionsgemisch, das neben 0,8 Mol.-% MSA noch $CO_2$ und CO neben Wasserdampf und Luft enthält. Das entspricht einer Ausbeute von 47 %. Eine Prüfung ergab, daß die Konzentrationsdifferenzen im Porengefüge des Wabenkörpers bei den genannten Versuchen vernachlässigbar gering sind.

**Ansprüche**

1. Verfahren zur Herstellung von Maleinsäureanhydrid durch partielle Oxidation von $C_4$-Kohlenwasserstoffen wie 1-Buten, 2-Buten und Butan oder Gemischen derselben in der Gasphase in Gegenwart von Phosphor-Vanadium-Sauerstoffkatalysatoren, dadurch gekennzeichnet, daß man Katalysatoren anwendet, die zuerst durch Imprägnieren chemisch inerter, abriebfester Trägerstoffe mit einer alkoholischen Lösung von Vanadinoxiden und/oder Estern der Vanadinsäure und/oder Alkoxiden des Vanadiums und/oder Vanadylalkoholaten und Phosphorsäureestern behandelt und darauf vor der üblichen Trocknung und Calzinierung der Einwirkung von Wasserdampf ausgesetzt worden sind.

2. Verfahren nach Anspruch 1, dadurch gekennzeichnet, daß man die Oxidation in der Wirbelschicht ausführt und als abriebfesten Träger Cordierit verwendet.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US-A-4 351 773 (E.C. MILBERGER et al.)<br>* Anspruch 1; Zusammenfassung * | 1,2 | C 07 D 307/60<br>C 07 C 51/215 |
| A | US-A-4 400 306 (D.E. DRIA et al.)<br>* Ansprüche 1, 2; Zusammenfassung * | 1,2 | |
| A | US-A-4 388 221 (E.L. MOOREHEAD)<br>* Ansprüche 10, 17, 19, 31; Zusammenfassung * | 1 | |
| A | US-A-4 336 198 (S.R. DOLHYJ et al.)<br>* Anspruch 1 * | 1 | |
| A | DE-A-2 437 154 (UCB S.A.)<br>* Ansprüche 1, 4, 5 * | 1 | RECHERCHIERTE SACHGEBIETE (Int. Cl.4) |
| D,A | US-A-4 225 465 (N.J. BREMER)<br>* Beispiele * | 1 | C 07 C 51/00<br>C 07 C 57/00<br>C 07 D 307/00 |
| A | US-A-4 515 899 (G.T. CLICK et al.)<br>* Beispiel 1 * | 1 | |

-----

Der vorliegende Recherchenbericht wurde für alle Patentansprüche erstellt.

| Recherchenort | Abschlußdatum der Recherche | Prüfer |
|---|---|---|
| BERLIN | 28-10-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82